(19) Europäisches Patentamt
European Patent Office
Office européen des brevets

(11) **EP 1 881 854 B1**

(12) **EUROPÄISCHE PATENTSCHRIFT**

(45) Veröffentlichungstag und Bekanntmachung des Hinweises auf die Patenterteilung:
**08.08.2012   Patentblatt 2012/32**

(21) Anmeldenummer: **06742358.2**

(22) Anmeldetag: **17.05.2006**

(51) Int Cl.:
***A61L 27/36*** (2006.01)

(86) Internationale Anmeldenummer:
**PCT/DE2006/000850**

(87) Internationale Veröffentlichungsnummer:
**WO 2006/122533 (23.11.2006 Gazette 2006/47)**

(54) **BIOARTIFIZIELLES HERZGEWEBETRANSPLANTAT UND VERFAHREN ZU SEINER HERSTELLUNG**

BIOARTIFICIAL HEART TISSUE GRAFT AND METHOD FOR THE PRODUCTION THEREOF

ELEMENT DE TRANSPLANTATION DE TISSU CARDIAQUE BIO-ARTIFICIEL, ET PROCEDE POUR LE REALISER

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **18.05.2005   DE 102005023599**

(43) Veröffentlichungstag der Anmeldung:
**30.01.2008   Patentblatt 2008/05**

(73) Patentinhaber: **Corlife GbR**
**30625 Hannover (DE)**

(72) Erfinder:
• **HAVERICH, Axel**
  **30916 Isernhagen (DE)**
• **LICHTENBERG, Artur**
  **69126 Heidelberg (DE)**
• **CEBOTARI, Serghei**
  **30627 Hannover (DE)**
• **TUDORACHE, Igor**
  **30625 Hannover (DE)**

(74) Vertreter: **Lins, Martina et al**
**Gramm, Lins & Partner GbR**
**Theodor-Heuss-Strasse1**
**38122 Braunschweig (DE)**

(56) Entgegenhaltungen:
WO-A-02/49681         WO-A-03/039610
DE-B3- 10 258 121     US-A- 5 215 541

EP 1 881 854 B1

## Beschreibung

Die Erfindung betrifft ein bioartifizielles Herzgewebetransplantat und ein Verfahren zu seiner Herstellung.

Unter einem bioartifiziellen Transplantat verstehen wir hier ein für eine Transplantation in Ersatz eines natürlichen Organs, Organteils oder Gewebes am Menschen vorgesehenes Körpergewebe aus allogener oder xenogener Quelle, das durch Entfernen insbesondere der darauf befindlichen Zellen, aber auch anderer Komponenten, wie Proteinen, Blutteilen und sonstigen immunogenen Komponenten für eine Implantation in einem anderen Körper geeignet gemacht wurde und so ein ursprünglich biologisches, aber manipuliertes und daher bioartifizielles Produkt darstellt. Das bioartifizielle Produkt kann ausschließlich seiner individuellen Spezifität beraubt sein, nämlich in erster Linie azellularisiert sein, oder es kann bereits individuell für den vorgesehenen Empfänger angepasst sein, z. B. durch in vitro Besiedlung mit empfängergeeigneten oder empfängereigenen Zellen, oder auch durch Aufbringen einer besonderen Beschichtung oder Behandlung, die den Einbau beim Empfänger erleichtern soll.

Im vorliegenden Fall handelt es sich bei dem bioartifiziellen Produkt um ein bioartifizielles Herzgewebetransplantat.

Aus dem Stande der Technik sind zahlreiche Verfahren zur Azellularisierung (auch Dezellularisierung genannt) biologischer Gewebe bekannt. Diese Verfahren dienen im Allgemeinen dem Zweck, biologische Transplantate für den Empfänger besser geeignet zu machen. Bei der Transplantation von Organen und Geweben, die von Organspendern stammen (allogene Transplantate, Homografts) oder tierischen Ursprungs sind (Xenotransplantate), kommt es zu Immunabstoßungsreaktionen, die schlimmstenfalls zum Verlust des Organs oder Gewebes führen können. Der Transplantatempfänger muss deshalb lebenslang Immunsuppressiva einnehmen, die eine solche Abstoßungsreaktion verhindern.

Um die Abstoßung der Transplantate grundsätzlich auszuschalten, wird seit langer Zeit versucht, das dafür bereitgestellte allogene oder auch xenogene Material für den Empfänger vorzubereiten, indem zunächst alle immunogenen Komponenten einschließlich der in den Geweben vorhandenen empfängerfremden Zellen entfernt werden. Da dies alle lebenden Zellen umfasst, sollte es gleichzeitig wirkungsvoll Kontaminationen durch Bakterien und Viren beseitigen können, was den Einsatz von Transplantaten tierischen Ursprungs vielfach erst ermöglicht. Nach der Entfernung dieser Zellen und sonstigen Komponenten bleibt in der Regel nur die extrazelluläre Matrix des Gewebes oder Organs erhalten, die aus den Hauptkomponenten, Kollagen, Fibrin und/oder Elastin besteht und weitere strukturgebende Substanzen wie Hyaluronine und Proteoglycane enthalten kann. Die extrazelluläre Matrix wird auch als interstitielles Gewebe oder interstitielles Bindegewebe bezeichnet.

In frühen Versuchen wurden die azellularisierten Gewebe vor der Transplantation zwischenkonserviert, beispielsweise kryokonserviert oder mit Glutaraldehyd fixiert. Dies führte jedoch zu Kalzifizierungen und anderen schwerwiegenden Nachteilen beim natürlichen Ein- und Umbau des Gewebes im Empfänger.

Man ging daher bald danach dazu über, die azellularisierten Konstrukte weiter für den Empfänger vorzubereiten, indem die entfernten Zellen durch andere ersetzt werden sollten, bevorzugt durch empfängereigene in vitro vermehrte Zellen des Empfängers von für das jeweilige bioartifizielle Transplantat geeigneter Art. Das größte Problem besteht dabei darin, das Transplantat naturähnlich wiederzubesiedeln, da in der Natur komplexe lokale Strukturen und eine Vielzahl von Zelltypen beteiligt sein können. Es wurde jedoch beobachtet, dass vorbesiedelte Transplantate im Körper des Transplantatempfängers umgebaut werden, so dass eine ein oder mehrschichtige Anfangsbesiedlung bestimmter Zellen ausreichend sein kann, wie beispielsweise in US 6 652 583 (Hopkins), US 5 843 182 (Goldstein) und US 5 899 936 (Goldstein) beschrieben. Dennoch kann auch ein rebesiedeltes Transplantat zu Problemen führen, wenn z.B. ein zu starkes Überwachsen des Transplantats mit körpereigenen Zellen erfolgt, so dass die geometrischen und mechanischen Eigenschaften zu stark beeinflusst werden (Hyperplasie).

Grundlage für die Konstruktion bioartifizieller Gewebetransplantate bleibt weiterhin eine wirkungsvolle Azellularisierung natürlichen biologischen Materials. Die Azellularisierung kann eine mechanische und/oder chemische Entfernung der Zellen vorsehen. Bei einer mechanischen Behandlung kann im Allgemeinen die extrazelluläre Matrix nicht geschont werden, so dass sich mechanische Behandlungsschritte auf die Entfernung außen anhaftender Zellen oder Membrane beschränken sollten. Chemische Azellularisierungsverfahren überwiegen daher bei weitem. Als chemische Mittel zum Ablösen, bzw. Verdauen oder Lysieren der Zellen werden unter anderem alkalische Lösungen, Enzyme, Glycerin, nicht-ionische und ionische Detergenzien verwendet, und zwar einzeln und in verschiedensten Kombinationen.

Durch die Anwendung bestimmter Mittel in bestimmter Reihenfolge erhofft man sich ganz bestimmte Effekte.

So schreibt beispielsweise die US 6 448 076 (Dennis et al) für die Azellularisierung speziell von Nerven vor, das Nervenpräparat zunächst in Glyzerin einzulegen, um die Zellmembranen aufzureißen und sodann intrazelluläre Proteine durch Einlegen in wenigstens eine Detergenzlösung zu denaturieren und zu entfernen.

Aus der US 5 215 541 A (Nashef) ist ein Verfahren zur Behandlung von biologischem Gewebe bekannt, bei dem das Gewebe mit Natriumdeoxycholat behandelt wird und hinterher implantiert werden kann.

Aus der DE 102 58 121 B ist ein Verfahren zur Herstellung von Bioprothesen bekannt, bei dem eine Detergenzbehandlung mit Desoxycholsäure und mit daran anschließenden Waschschritten in physiologischer Kochsalzlösung durchgeführt wird.

Aus dem US-Patent 6 376 244 ist es bekannt, eine dezellularisierte Nierenmatrix zu erzeugen, indem das Nierenpräparat zunächst in destilliertes Wasser eingelegt wird, um die Zellmembranen zu zerstören, und danach zelluläres Material mit alkalischer Detergenzlösung extrahiert wird.

Gemäß US 6 734 018 ist zur Herstellung eines Weichgewebe-Transplantats ein Behandeln des Gewebes mit einer Extraktionslösung, einer Behandlungslösung und einer Waschlösung vorgesehen, wobei die Extraktionslösung eine alkalische Lösung mit einem nicht-ionischen Detergenz und wenigstens einer Endonuclease ist und die Behandlungslösung ein anionisches Detergenz enthält. Die Behandlung mit anionischem Detergenz dient dort nicht zur Azellularisierung, sondern gleichzeitig zur Behandlung des Gewebes, nämlich zur Beeinflussung der Wiederbesiedlung. Dabei wird ausgeführt, dass eine Behandlung mit einem starken anionischen Tensid zu Wechselwirkungen mit den Matrixproteinen und zur Anlagerung des Tensids (oder Detergenz) in dem azellularisierten Gewebe führen kann, so dass das anionische Detergenz in der Matrix zurückgehalten und bis nach der Transplantation in dem Gewebe erhalten bleiben kann. Dies ist wegen damit verbundener toxischer Effekte einerseits unerwünscht, in Maßen jedoch auch erwünscht, um so die Rebesiedlungsgeschwindigkeit zu modulieren, die durch die Anwesenheit des Detergenzes verlangsamt wird.

In US 6 734 018 wird auch bereits ausführlicher auf die Bedeutung und Wichtigkeit der Schrittreihenfolge eingegangen. So wird ausgeführt, dass die Behandlung mit SDS vor einer Behandlung mit Salzlösung zu anderen Ergebnissen führt als die Behandlung mit SDS nach einer Behandlung mit Salzlösung.

Der Erfindung liegt die Aufgabe zugrunde, ein schonendes und effizientes Verfahren zur Herstellung einer stabilen, funktionell intakten und nach Transplantation möglichst haltbaren azellularisierten Herzgewebematrix zu schaffen. Dabei soll angestrebt werden, dass eine Transplantation mit und ohne Rebesiedlung mit patienteneigenen Zellen möglich ist.

Zur Lösung dieser Aufgabe sieht die Erfindung bei einem Verfahren zur Herstellung eines bioartifiziellen Herzgewebe-Transplantats, bei welchem in und/oder an einer extrazellulären Matrix anhaftende biologische Zellen von einem Herzgewebepräparat, insbesondere einer Herzklappe oder einem Herzgefäß, in vitro entfernt werden, vor, dass das Verfahren folgende Schritte in der genannten Reihenfolge aufweist:

a) Bereitstellen des Herzgewebepräparats natürlichen Ursprungs;

b) Entfernen in dem Gewebe befindlicher Zellen von der extrazellulären Matrix mit Hilfe einer Azellularisierungslösung aus einer wässrigen Lösung wenigstens eines starken anionischen Detergenzes, die mindestens Natriumdeoxycholat enthält;

c) osmotisches Behandeln des Gewebes mit destilliertem oder entionisiertem Wasser;

d) Behandeln des Gewebes mit physiologischer Salzlösung.

Es wurde gefunden, dass die Einhaltung gerade dieser Schritte, deren Bedeutung nachfolgend noch erläutert wird, insbesondere bei der Azellularisierung von Herzgeweben besonders gute Resultate liefert. Durch das Verfahren werden azellularisierte bioartifizielle Transplantate erhalten, die gute mechanische und physiologische Eigenschaften besitzen. Die Transplantate versprechen aufgrund bisher durchgeführter Versuche, auch ohne vorherige in vitro Besiedlung im Körper gut angenommen zu werden und lassen aufgrund ihrer biomechanischen Eigenschaften eine lange Haltbarkeit erwarten. Für die Anwendung gerade bei Herzklappen ist besonders wichtig, dass die Basalmembran bei dem Verfahren intakt bleibt. Allgemein entspricht die Morphologie in einem hohen Maße der natürlichen Morphologie des zugrundeliegenden Gewebepräparats, und es kann eine hohe Zellfreiheit erzielt werden.

Der erste Verfahrensschritt eines Azellularisierungsverfahrens umfasst immer die Bereitstellung eines natürlichen Gewebes, das allogenen oder xenogenen Ursprungs sein kann. Falls erforderlich wird das natürliche Gewebestück so präpariert, wie es für die Transplantation bei dem potentiellen Empfänger am besten geeignet ist. Es gibt jedoch auch solche Präparate, die für eine Gruppe von Transplantatempfängern immer geeignet sind, z. B. Herzklappen in einem bestimmten Ringgrößenbereich. Der Gefäßansatz um den Klappenring kann unterschiedlich lang ausgebildet sein, bzw. zugeschnitten werden. Schritt a) des Verfahrens umfasst daher das Aussuchen und Zuschneiden des natürlichen Präparates, ggf. auch nach Zwischenlagerung, und das Einbringen in eine für das Verfahren geeignete Apparatur oder ein geeignetes Gefäß, z. B. eine Schale oder Flasche.

Vorzugsweise handelt es sich bei dem Herzgewebepräparat um eine Pulmonalklappe (Valva truncti pulmonalis), eine Aortenklappe (Valva aortae), eine Trikuspidalklappe (Valva atrioventricularis dextra), eine Mitralklappe (Valva atrioventricularis sinistra), ein klappenloses Gefäßstück mit oder ohne Verzweigung oder ein Herzbeutelgewebestück für ein Herzgewebepatch.

Die im nächsten Schritt des Verfahrens verwendete Azellularisierungslösung (Schritt b) enthält erfindungsgemäß starke anionische Detergenzien und hierunter in jedem Fall Natriumdeoxycholat, da die Verwendung dieses Detergenzes sich für die Azellularisierung in dieser Verfahrensstufe und in Zusammenhang mit den übrigen Schritten als besonders wirkungsvoll herausgestellt hat.

Neben Natriumdeoxycholat sollte wenigstens ein weiteres anionisches Detergenz aus der Gruppe bestehend aus Salzen höherer aliphatischer Alkohole, vorzugsweise deren Sulfaten und Phosphaten, sulfonierten Alkanen und sulfonierten Alkylarenen, jeweils mit 7 bis 22 Kohlenstoffatomen in vorzugsweise nicht-verzweigter Kette, enthalten sein, da überraschenderweise gefunden wurde, dass sich diese Mischungen aus dem Gewebe leichter auswaschen lassen als die genannten Mittel einzeln.

Besonders bevorzugt ist derzeit, dass die Azellularisierungslösung neben Natriumdeoxycholat Natriumdodecylsulfat (SDS) enthält, vorzugsweise beide Komponenten in einer Konzentration zwischen 0,05 und 3 Gew.-%, zusammen nicht über 5 Gew.-% und in besonders bevorzugter Ausführungsform in Konzentrationen zwischen 0,3 und 1 Gew.-%, besonders bevorzugt je 0,5 Gew.-%.

In einer solchen bevorzugten Lösung von beispielsweise 0,5 Gew.-% SDS und 0,5 Gew.-% Natriumdeoxycholat in Wasser wird das natürliche Gewebe wie an sich aus anderen Azellularisierungsverfahren grundsätzlich bekannt behandelt. Vorzugsweise kann das natürliche Gewebe für ca. 24 Stunden, je nach Gewebe aber auch länger oder kürzer, bei Raumtemperatur oder leicht reduzierter Raumtemperatur, etwa bei 15 bis 30 °C, unter Schütteln oder Schwenken in einem Behälter mit oder ohne Einspannvorrichtung für das Gewebe behandelt werden. Das Gewebe soll dabei vollständig von der Azellularisierungslösung bedeckt sein. Die Azellularisierungszeit kann der Fachmann mit Hilfe von Vorversuchen an das jeweils zu behandelnde Gewebe anpassen, auch kann die Azellularisierungslösung mehrfach gewechselt werden, falls dies bei der jeweiligen Azellularisierungsaufgabe vorteilhaft erscheint.

Für diesen wie für die anderen Behandlungsschritte gilt, dass im Wesentlichen alle im Stande der Technik üblichen Vorgehensweisen angewendet werden können, d.h. Einlegen in den jeweiligen Lösungen in Schalen oder Behältern, Behandeln in besonderen Bioreaktoren, teils auch unter pulsatilem Druck, Spülen mit einer über die Behandlungsdauer im Kreislauf geführten Lösung, usw.. Die Lösungen können je nach Bedarf ein- oder mehrfach ausgetauscht werden. Selbstverständlich wird unter sterilen Bedingungen gearbeitet.

Im nächsten Verfahrensschritt (Schritt c) erfolgt eine osmotische Behandlung des Gewebes für im Allgemeinen wenigstens 20 Stunden mit destilliertem oder entionisiertem Wasser. Dieser Schritt hat sich überraschenderweise im Zusammenhang des Gesamtverfahrens als vorteilhaft herausgestellt, obwohl ein Quellen der Matrix, wie es in destilliertem oder entionisiertem Wasser zwangsweise erfolgt sonst nicht als vorteilhaft angesehen wird. Die Behandlung mit destilliertem Wasser ist innerhalb von Azellularisierungsverfahren grundsätzlich bekannt, jedoch sonst für die Lyse der Zellen, d.h. der Zellmembranen, die hier bereits im vorausgegangenen Schritt größtenteils entfernt worden sind. Bei der Erfindung erfolgt also in diesem Schritt eine Behandlung der extrazellulären Matrix, die hierdurch offenbar - im Zusammenhang mit den übrigen Schritten - in einen positiven Zustand versetzt wird.

Im letzten Schritt (Schritt d) des Verfahrens wird das Gewebe mit physiologischer Salzlösung, und zwar vorzugsweise unter kontinuierlichem Durchlauf oder unter wenigstens 3maligem Auswechseln der Spüllösung behandelt. Unter einer physiologischen Salzlösung wird hier eine im Wesentlichen isotonische Lösung verstanden, bei der es sich insbesondere um eine gepufferte Salzlösung handeln kann. Vorzugsweise wird PBS oder physiologische Kochsalzlösung verwendet.

Das Spülen vorzugsweise mit PBS erfolgt im Allgemeinen durch Einlegen des Präparats in diese Lösung und Schütteln oder Schwenken bei 15 bis 30 °C bzw. Raumtemperatur über vorzugsweise 72 bis 96 Stunden. Dabei sollte die Lösung mindestens 3mal, vorzugsweise aber etwa 6 bis 8mal, beispielsweise 7mal gewechselt werden. Es kann alternativ auch ein kontinuierliches Spülen mit PBS im Durchlauf erfolgen.

Das Spülen sollte solange durchgeführt werden, bis in der Spüllösung keine oder eine nicht mehr zelltoxische Restkonzentration an Detergenz gemessen wird.

Der Salzlösung in Schritt d) können auch bei derartigen Verfahren übliche Zusätze, wie Antibiotika und/oder Antimykotika zugegeben sein.

Im Anschluss an das Verfahren kann das bioartifizielle Herzgewebetransplantat eingesetzt werden. Bis zur Verwendung kann es einige Zeit kühl gelagert werden, wobei es sich in isotonischer Lösung befinden sollte. Das Transplantat kann auch kryokonserviert werden, falls eine unmittelbare Verwendung nicht möglich ist.

Das Verfahren wird im Weiteren anhand von Versuchsbeispielen erläutert werden, wobei eine Übersicht zu den biomechanischen Eigenschaften in Tabelle 1 gegeben wird. Die biomechanischen Eigenschaften unterscheiden sich deutlich von denen, die mit in der Schrittfolge oder der Zusammensetzungen der Lösungen abweichenden Verfahren erzielt werden können.

Das bioartifizielle Herzgewebetransplantat kann so, wie nach dem erfindungsgemäßen Verfahren erhalten, implantiert werden. Nach derzeitigem Wissensstand wird angenommen, dass die Azellularisierung allein oder zumindest im Wesentlichen mit anionischem Detergenz gerade eine Herzgewebematrix schonend zu dezellularisieren vermag, wobei die Matrixproteine sich in einem solchen Ladungszustand befinden, der optimal für eine Wiederbesiedlung im Körper des Empfängers oder in vitro zu sein scheint, ohne dass noch zelltoxische Konzentrationen an tensidischem Azellularisierungsmittel in der Matrix vorhanden wären. Auch die biomechanischen Eigenschaften sind geeignet, dass eine unmittelbare Implantation des noch unbesiedelten Herzgewebetransplantats möglich ist.

Das azellularisierte Herzgewebepräparat kann aber auch vor der Transplantation in vitro mit lebensfähigen biologischen Zellen re-besiedelt werden, vorzugsweise mit Endothelzellen. Die für eine Rebesiedlung in vitro verwendeten Zellen sind vorzugsweise autologe Zellen des potentiellen Transplantatempfängers, die ihm in Vorbereitung der Transplantation entnommen und in vitro vermehrt wurden. Die hierzu erforderlichen Verfahren sind im Stande der Technik bekannt.

Beispiel:

Eine Pulmonalklappe (Valva truncti pulmonalis) mit Gefäßkonduit wurde einem porcinem Herzen entnommen und zur Entfernung von Blutresten mit PBS-Lösung gespült. Zur Azellularisierung erfolgte eine 24stündige Inkubation in einer 0,5% Natriumcholat / 0,5% SDS-Lösung bei 20 °C unter Schütteln. Danach wurde das Gewebe für 24 Stunden mit destilliertem Wasser bei 20 °C unter Schütteln inkubiert. Abschließend wurde das Gewebe für 96 Stunden bei 20 °C unter Schütteln in PBS-Lösung gespült. Bei diesem Vorgang wurde die PBS-Lösung alle 12 Stunden gewechselt.

Biomechanische Testungen:

Proben porciner Pulmonalklappenwände (5 Proben pro Untersuchungsgruppe) wurden auf eine Länge und Breite von 15 mm mal 10 mm zugeschnitten und so in speziell dafür konstruierten Klammern gehaltert, dass die unbelastete Referenzlänge des unter seinem eigenen Gewicht hängenden Probestücks 10 mm betrug. Die Querschnittsfläche entlang der so definierten Referenzlänge wurde mit einem kontaktfreien Lasermicrometer (LDM-303H-SP, Takikawa Engineering Co., Tokyo, Japan) bestimmt. Die Proben wurden für eine Testung in Längs- und Querrichtung in Bezug auf die Gefäßrichtung (Arteria Pulmonaris) zugeschnitten und während der Testung mit PBS-Lösung feucht gehalten.
Die Probestücke wurden dann mit 0,01 Newton vorbelastet und nach und nach bis zum makroskopischen Versagen gelängt. Dieser Schritt erfolgte in einer Materialtestungs-Apparatur (Modell 1445, Zwick GmbH, Ulm, Deutschland) bei einer Geschwindigkeit von 0,1 mm Dehnung pro Sekunde. Es wurden für jedes getestete Probestück Kraft-Auslängungs- und Spannungs-Dehnungs-Kurven aufgenommen und die Grenzspannung, strukturelle Steifigkeit, Grenzdehnung des Probestücks (Bruchdehnung) und das Elastizitätsmodul (Young's Modul) bestimmt. Die Struktursteifigkeit und das Elastizitätsmodul wurden im linearen Bereich der Kraft-Auslängungs- bzw. Spannungs-Dehnungs-Kurven bestimmt. Die Grenzspannung und die Reißkraft wurden an dem Punkt genommen, an dem der erste signifikante Abfall der Zugspannung bzw. der Kraft zu erkennen war (Tabelle 1).
Definition der in Tabelle 1 angegebenen Messparameter:

Reißkraft $F_R$[N]:

Als Reißkraft bezeichnet man die gemessene Kraft im Augenblick des Reißens. Die Reißkraft wurde an dem Punkt genommen, an dem der erste signifikante Abfall der Kraft zu erkennen war.

Reißfestigkeit $\delta_R$ [N/mm$^2$]:

Die Reißfestigkeit ist der Quotient aus der im Augenblick des Reißens gemessenen Kraft $F_R$ und dem Anfangsquerschnitt $A_0$[N/mm$^2$] [entspricht MPa]

$$\delta_R = F_R / A_0[\text{N/mm}^2] \text{ [entspricht MPa]}$$

Elastizitätsmodul E [N/mm$^2$]:

Der Elastizitätsmodul (E-Modul) ist ein Maß für die Festigkeit eines Materials. Er gibt an, um wie viel sich ein Stoff unter einer bestimmten Belastung dehnt.
Der E-Modul ist definiert als die Steigung im Graphen der Spannungs-DehnungsKurve innerhalb des Elastizitätsbereichs. Bei linearem Verlauf der Spannungs-Dehnungs-Kurve (Proportionalitätsbereich) gilt:

$$E = \delta / \varepsilon \text{ [N/mm2] [entspricht MPa]}$$

Dabei bezeichnet $\delta$ die (Zug-)Spannung und $\varepsilon$ die Dehnung.

**Tabelle 1**

| | Vergleichsgruppe, n=5 | | Trypsion Gruppe, n=5 | | NaD Gruppe, n=5 | | NaD+SDS Gruppe, n=5 | | SDS Gruppe, n=5 | |
|---|---|---|---|---|---|---|---|---|---|---|
| **Ergebnisse der biomechanischen Testung** | | | | | | | | | | |
| | Long | trans | long | trans | long | trans | long | trans | long | trans |
| Fläche (mm$^2$) | 29,2±5,7 | 29,6±6,3 | 27,6±5,4 | 28,1±5,9 | 31,4±5,4 | 26,6±5,8 | **31,8±6,6** | **29,9±1,6** | 26,3±4,0 | 26,8±4,88 |
| Grenzkraft (N) | 9,5±4,3 | 13,9±1,5 | 5,0±2,37* | 11,8±3,2 | 9,8±1,3 | 16,2±5,6 | **6,6±2,1** | **11,4±3,9** | 7,9±5,6 | 15,5±5,8 |
| Steifigkeit (N/mm) | 3,0±2,6 | 4,3±1,6 | 2,3±1,5 | 4,2±1,5 | 3,0±0,79 | 7,0±3,0 | **2,7±0,7** | **3,9±1,25** | 2,6±1,4 | 5,3±1,5 |
| Bruchdehnung (mm/mm) | 0,8±0,48 | 1,6±0,63 | 0,4±0,2* | 0,5±0,2† | 0,5±0,1 | 1,0±0,3 | **0,6±0,2** | **1,2±0,08** | 0,7±0,5 | 0,7±0,4* |
| Grenzbelastung (MPa) | 0,32±0,15 | 0,50±0,18 | 0,18±0,06* | 0,43±0,16 | 0,32±0,05 | 0,61±0,2 | **0,22±0,11** | **0,39±0,14** | 0,29±0,11 | 0, 57±0,13 |
| Young's-Modul (MPa) | 1,04±0,94 | 0,86±0,57 | 0,69±0,5 | 1,69±1,04 | 0,99±0,25 | 1,75±1,35 | **0,90±0,38** | **1,18±0,55** | 0,97±0,52 | 2,06±0,84 * |
| Daten angegeben als Mittelwert ± Standardwaschung <br> *p < 0,05, †p < 0,01, §p < 0,001 gegen Vergleichsgruppe | | | | | | | | | | |

Die Testergebnisse werden anhand der Abbildungen verdeutlicht. Es zeigen:

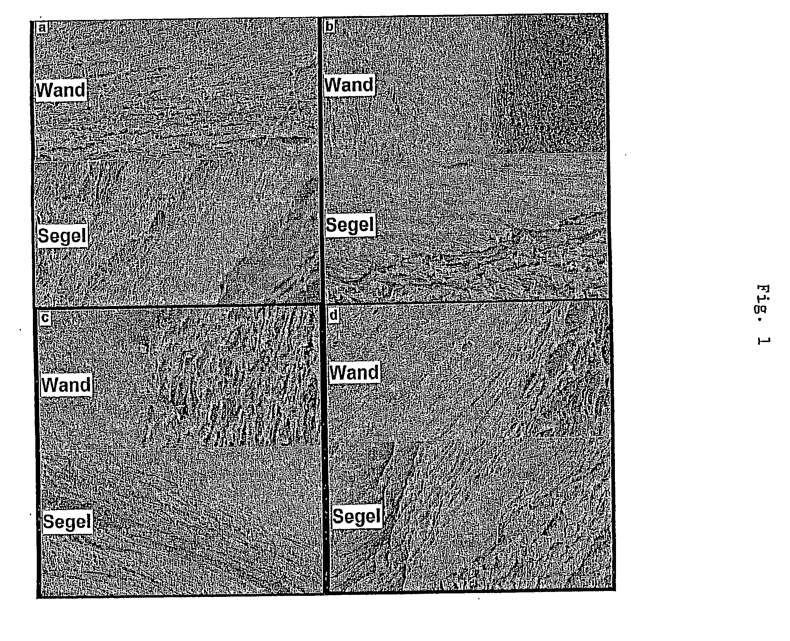

Abb.1a) mit Trypsin-Lösung (0,05% Trypsin/0,02% EDTA) behandelte Klappe;
Abb. 1b) mit Natriumdeoxycholat-Lösung/(1%ig) behandelte Klappe;
Abb. 1c) mit Natriumdeoxycholat und SDS (je 0,5%ige Lösungen) behandelte Klappe
Abb. 1 d) mit SDS-Lösung (1 %ig) behandelte Klappe
Abb. 2) Spannungs-Dehnungs-Kurven (oben) und Kraft-Auslängungskurven (unten).

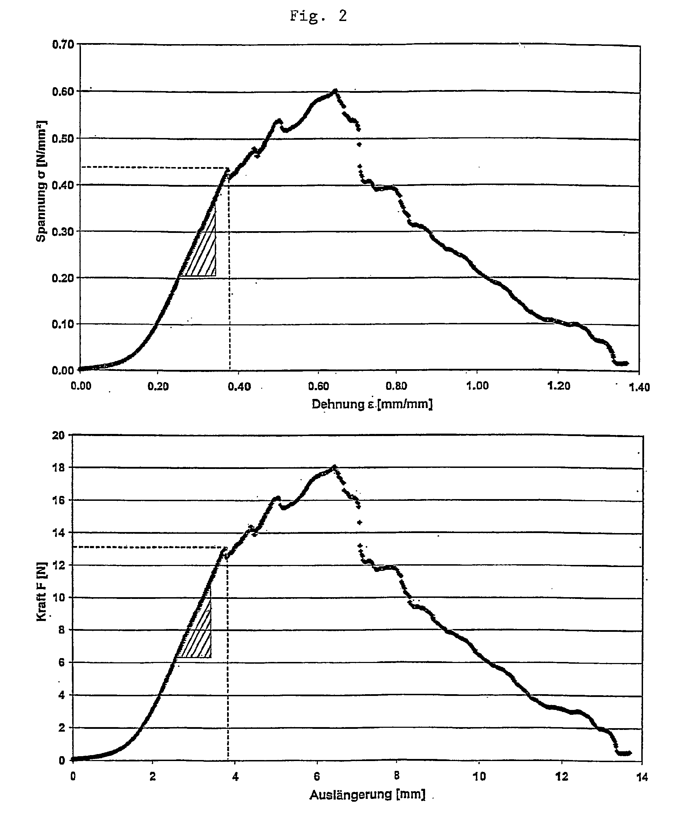

Die Abbildungen 1a) bis 1d) zeigen rasterelektronenmikroskopische Aufnahmen mit verschiedenen Azellularisierungslösungen (bei sonst gleichem Verfahren) behandelter porciner Pulmonalklappenbereiche. Gezeigt ist jeweils eine Intraluminaloberfläche mit Transversalschnitt von Wand (im Bild jeweils oben) und Segel (im Bild jeweils unten).
Wie zu erkennen ist, schädigt die Trypsinbehandlung die Basalmembran stark. Mit dem erfindungsgemäßen Verfahren und Natriumdeoxycholat in der Azellularisierungslösung ergeben sich augenscheinlich gute Ergebnisse.
Die biomechanischen Eigenschaften zu diesen Versuchen sind in Tabelle 1 quantifiziert. Typische Spannungs-Dehnungs-Kurven und Kraft-Auslängungskurven sind in Abb. 2 gezeigt. Die schraffierten Steigungsdreiecke kennzeichnen die Bereiche, in denen der Elastizitätsmodul und die strukturelle Steifigkeit berechnet werden. Der mit gestrichelten Linien gekennzeichnete Kurvenpunkt kennzeichnet die Grenzspannung, die Grenzdehnung und die Reißkraft.
Es zeigt sich, dass die Steifigkeit in Longitudinal- und Transversalrichtung gegenüber nativen Vergleichsgeweben um nicht mehr als etwa +- 15% verändert ist. Auch das Young-Modul ist gegenüber nativen Vergleichsgeweben nur moderat erhöht. Die biomechanischen Eigenschaften des Verfahrensprodukts können daher als insgesamt gut bezeichnet werden.

## Patentansprüche

1. Verfahren zur Herstellung eines bioartifiziellen Herzgewebetransplantats, bei welchem in und/oder an einer extrazellulären Matrix anhaftende biologische Zellen von einem Herzgewebepräparat, insbesondere einer Herzklappe oder einem Herzgefäß, in vitro entfernt werden, wobei das Verfahren folgende Schritte in der genannten Reihenfolge aufweist:

   a) Bereitstellen des Herzgewebepräparats natürlichen Ursprungs;
   b) Entfernen in dem Gewebe befindlicher Zellen von der extrazellulären Matrix mit Hilfe einer Azellularisierungslösung aus einer wässrigen Lösung wenigstens eines starken anionischen Detergenzes, die mindestens Natriumdeoxycholat enthält;
   c) osmotisches Behandeln des Gewebes mit destilliertem oder entionisiertem Wasser;
   d) Behandeln des Gewebes mit physiologischer Salzlösung.

2. Verfahren nach Anspruch 1, **dadurch gekennzeichnet, dass** das bereitgestellte Herzgewebepräparat eine Pulmonalklappe (Valva truncti pulmonalis), eine Aortenklappe (Valva aortae), eine Trikuspidalklappe (Valva atrioventricularis dextra), eine Mitralklappe (Valva atrioventricularis sinistra), ein klappenloses Gefäßstück mit oder ohne Verzweigung oder ein Herzbeutelgewebestück für ein Herzgewebepatch, jeweils allogenen oder xenogenen Ursprungs ist.

3. Verfahren nach Anspruch 1 oder 2, **dadurch gekennzeichnet, dass** die Azellularisierungslösung neben Natriumdeoxycholat wenigstens ein weiteres anionisches Detergenz aus der Gruppe bestehend aus Salzen höherer aliphatischer Alkohole, vorzugsweise deren Sulfaten und Phosphaten, sulfonierten Alkanen und sulfonierten Alkylarenen, jeweils mit 7 bis 22 Kohlenstoffatomen in vorzugsweise nicht-verzweigter Kette, enthält.

4. Verfahren nach einem der Ansprüche 1 bis 3, **dadurch gekennzeichnet, dass** die Azellularisierungslösung neben Natiumdesoxycholat Natriumdodecylsulfat (SDS) enthält, vorzugsweise beide Komponenten in einer Konzentration zwischen 0,05 und 3 Gew.-%, zusammen nicht über 5 Gew.-%, weiter vorzugsweise zwischen 0,3 und 1 Gew.-%, besonders bevorzugt je 0,5 Gew.- %.

5. Verfahren nach einem der Ansprüche 1 bis 4, **dadurch gekennzeichnet, dass** das Verfahren bei Temperaturen zwischen 15 und 30 °C durchgeführt wird.

6. Verfahren nach einem der Ansprüche 1 bis 5, **dadurch gekennzeichnet, dass** für die Behandlungsschritte a) bis d) das Gewebes in einem Behälter mit oder ohne Einspannvorrichtung für das Gewebepräparat in die entsprechende Behandlungslösung eingelegt und vollständig mit dieser bedeckt wird, wobei der Behälter vorzugsweise während der Behandlungsdauer oder Teilen der Behandlungsdauer geschüttelt oder geschwenkt wird.

7. Verfahren nach einem der Ansprüche 1 bis 6, **dadurch gekennzeichnet, dass** die Salzlösung in Schritt d) eine gepufferte Salzlösung, vorzugsweise PBS oder physiologische Kochsalzlösung ist.

8. Verfahren nach einem der Ansprüche 1 bis 7, **dadurch gekennzeichnet, dass** die Salzlösung in Schritt d) wenigstens 3mal, vorzugsweise wenigstens 6mal erneuert wird, oder dass unter kontinuierlichem Durchlauf der PBS-Lösung gearbeitet wird.

9. Verfahren nach einem der Ansprüche 1 bis 8, **dadurch gekennzeichnet, dass** der Salzlösung in Schritt d) Antibiotika und/oder Antimykotika zugesetzt sind.

**Claims**

1. A method for producing a bioartificial heart tissue graft in which biological cells adhering in and/or on an extracellular matrix are removed from a heart tissue specimen, in particular a heart valve or a cardiac vessel, in vitro, where the method includes the following steps in the stated sequence:

   a) provision of the heart tissue specimen of natural origin;
   b) removal of cells present in the tissue from the extracellular matrix with the aid of an acellularizing solution composed of an aqueous solution of at least one strong anionic detergent which comprises at least sodium deoxycholate;
   c) osmotic treatment of the tissue with distilled or deionized water;
   d) treatment of the tissue with physiological saline solution.

2. The method as claimed in claim 1, **characterized in that** the provided heart tissue specimen is a pulmonary valve (valva truncti pulmonalis), an aortic valve (valva aortae), a tricuspid valve (valva atrioventricularis dextra), a mitral valve (valva atrioventricularis sinistra), a valveless vessel piece with or without branch or a pericardial tissue piece for a heart tissue patch, in each case of allogeneic or xenogeneic origin.

3. The method as claimed in claim 1 or 2, **characterized in that**, besides sodium deoxycholate, the acellularizing solution comprises at least one further anionic detergent from the group consisting of salts of higher aliphatic alcohols, preferably sulfates and phosphates thereof, sulfonated alkanes and sulfonated alkylarenes, in each case having 7 to 22 carbon atoms in a preferably unbranched chain.

4. The method as claimed in any of claims 1 to 3, **characterized in that**, besides sodium deoxycholate, the acellularizing solution comprises sodium dodecyl sulfate (SDS), preferably both components in a concentration between 0.05 and 3 % by weight, together not more than 5 % by weight, further preferably between 0.3 and 1 % by weight, particularly preferably each 0.5 % by weight.

5. The method as claimed in any of claims 1 to 4, **characterized in that** the method is carried out at temperatures between 15 and 30 °C.

6. The method as claimed in any of claims 1 to 5, **characterized in that** for treatment steps a) to d) the tissue is put into the appropriate treating solution in a container with or without clamping device for the tissue specimen and is completely covered by this solution, with the container being shaken or swirled preferably during the treatment period or parts of the treatment period.

7. The method as claimed in any of claims 1 to 6, **characterized in that** the saline solution in step d) is a buffered saline solution, preferably PBS, or physiological sodium chloride solution.

8. The method as claimed in any of claims 1 to 7, **characterized in that** the saline solution in step d) is renewed at least 3 times, preferably at least 6 times, or **in that** the PBS solution flows through continuously.

9. The method as claimed in any one of claims 1 to 8, **characterized in that** antibiotics and/or antimycotics are added to the saline solution in step d).

**Revendications**

1. Procédé de fabrication d'un transplant de tissu cardiaque bioartificiel, dans lequel des cellules biologiques adhérant dans et/ou à une matrice extracellulaire d'une préparation de tissu cardiaque, en particulier une valvule cardiaque ou un vaisseau cardiaque, sont éliminées in vitro, le procédé présentant les étapes suivantes, dans l'ordre mentionné :

   a) mise à disposition de la préparation de tissu cardiaque d'origine naturelle ;
   b) élimination de cellules se trouvant dans le tissu de la matrice extracellulaire à l'aide d'une solution d'acellularisation constituée d'une solution aqueuse d'au moins un détergent anionique fort, qui contient au moins du désoxycholate de sodium ;
   c) traitement osmotique du tissu avec de l'eau distillée ou désionisée ;
   d) traitement du tissu par une solution saline physiologique.

2. Procédé selon la revendication 1, **caractérisé en ce que** la préparation de tissu cardiaque mise à disposition est une valve du tronc pulmonaire (Valva trunci pulmonalis), une valve aortique (Valva aortae), une valve tricuspide (Valva atrioventricularis dextra), une valve mitrale (Valva atrioventricularis sinistra), un morceau de veine sans valve, avec ou sans ramification ou un morceau du péricarde pour un patch cardiaque, à chaque fois d'origine allogène ou xénogène.

3. Procédé selon la revendication 1 ou 2, **caractérisé en ce que** la solution d'acellularisation contient, outre le désoxycholate de sodium, au moins un autre détergent anionique du groupe constitué par les sels d'alcools aliphatiques supérieurs, de préférence leurs sulfates et phosphates, les alcanes sulfonés et les alkylarènes sulfonés, comprenant à chaque fois 7 à 22 atomes de carbone dans la chaîne de préférence non ramifiée.

4. Procédé selon l'une quelconque des revendications 1 à 3, **caractérisé en ce que** la solution d'acellularisation contient, outre le désoxycholate de sodium, du dodécylsulfate de sodium (SDS), de préférence les deux composants en une concentration entre 0,05 et 3% en poids, ensemble à raison de pas plus de 5% en poids, plus préférablement entre 0,3 et 1% en poids, de manière particulièrement préférée chacun à 0,5% en poids.

5. Procédé selon l'une quelconque des revendications 1 à 4, **caractérisé en ce que** le procédé est réalisé à des températures entre 15 et 30°C.

6. Procédé selon l'une quelconque des revendications 1 à 5, **caractérisé en ce que** pour les étapes de procédé a) à d), le tissu est placé dans un récipient, avec ou sans dispositif de fixation pour la préparation de tissu, dans la solution de traitement correspondante et est complètement recouvert par celle-ci, le récipient étant de préférence agité ou pivoté pendant la durée de traitement ou des périodes de la durée de traitement.

7. Procédé selon l'une quelconque des revendications 1 à 6, **caractérisé en ce que** la solution de sel dans l'étape d) est une solution de sel tamponnée, de préférence du PBS, ou une solution physiologique de chlorure de sodium.

8. Procédé selon l'une quelconque des revendications 1 à 7, **caractérisé en ce que** la solution de sel est renouvelée au moins 3 fois, de préférence au moins 6 fois au cours de l'étape d) ou **en ce qu'**on travaille sous un passage continu de la solution de PBS.

9. Procédé selon l'une quelconque des revendications 1 à 8, **caractérisé en ce qu'**un/des antibiotique(s) et/ou antimycotique(s) est/sont ajouté(s) à la solution de sel dans l'étape d).

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6652583 B, Hopkins
- US 5843182 A, Goldstein
- US 5899936 A, Goldstein
- US 6448076 B, Dennis
- US 5215541 A, Nashef
- DE 10258121 B
- US 6376244 B
- US 6734018 B